# EUROPEAN PATENT SPECIFICATION

(11) **EP 2 210 601 B1**
(45) Date of publication and mention of the grant of the patent: **19.07.2017**
(21) Application number: 08843904.7
(22) Date of filing: 31.10.2008
(51) Int. Cl.: A61K 31/198, A23L 33/175, A61K 31/401, A61K 31/405, A61K 31/4172, A61P 3/02, A61P 43/00

(54) **ANTI-FATIGUE AGENT COMPRISING AMINO ACID COMPOSITION**
MITTEL GEGEN MÜDIGKEIT MIT EINER AMINOSÄURE-ZUSAMMENSETZUNG
AGENT ANTIFATIGUE COMPRENANT UNE COMPOSITION D'ACIDES AMINÉS

(30) Priority: 31.10.2007 JP 2007282970
(43) Date of publication of application: 28.07.2010
(73) Proprietor: MEIJI CO., LTD, Chuo-ku Tokyo 104-8306 (JP)
(72) Inventor: SAITO, Masato, Odawara-shi Kanagawa 2500862 (JP); ARITA, Hiroyuki, Odawara-shi Kanagawa 2500862 (JP); TAKAMURA, Masanori, Tokyo 136-8908 (JP)
(74) Representative: Nuss, Laurent
(86) International application number: PCT/JP2008/069938
(87) International publication number: WO 2009/057775

(56) References cited:
- WO-A1-2004/028528
- WO-A1-2007/145239
- WO-A1-2008/105368
- JP-A- 3 129 318
- JP-A- 2004 352 696
- JP-B2- 3 814 683
- US-A- 5 026 721
- US-B1- 6 224 861
- US-B1- 6 287 757
- DATABASE WPI Week 199128 Thomson Scientific, London, GB; AN 1991-203970 XP002092564, & JP 3 128318 A (NIPPON STEEL CORP) 31 May 1991 (1991-05-31)
- SMRIGA MIRO ET AL: "Exercise-dependent preference for a mixture of branched-chain amino acids and homeostatic control of brain serotonin in exercising rats", JOURNAL OF NUTRITION, vol. 136, no. 2, February 2006 (2006-02), pages 548S-552S, XP002614997, ISSN: 0022-3166
- OHTANI MASARU ET AL: "Amino acid mixture improves training efficiency in athletes", JOURNAL OF NUTRITION, vol. 136, no. 2, February 2006 (2006-02), pages 538S-543S, XP002614998, ISSN: 0022-3166
- DATABASE WPI Week 199508 Thomson Scientific, London, GB; AN 1995-057293 XP002614999, & JP 6 336426 A (DOKURITSU GYOSEI HOJIN RIKAGAKU KENKYUSH) 6 December 1994 (1994-12-06)
- SUGITA MASAAKI ET AL: "Effect of a selected amino acid mixture on the recovery from muscle fatigue during and after eccentric contraction exercise training.", BIOSCIENCE BIOTECHNOLOGY AND BIOCHEMISTRY, vol. 67, no. 2, February 2003 (2003-02), pages 372-375, XP002615000, ISSN: 0916-8451
- DATABASE WPI Week 199641 Thomson Scientific, London, GB; AN 1996-408309 XP002615001, & JP 8 198748 A (AJINOMOTO KK) 6 August 1996 (1996-08-06)
- KANDO KOBAYASHI: 'Sport to Amino-san Supplement' SHOKU NO KAGAKU vol. 255, 1999, pages 93 - 88, XP008141118

## Description

### [Technical Field]

The present invention relates to an anti-fatigue agent which comprises an amino acid composition consisting of specific amounts of specific kinds of amino acids.

### [Background Art]

Phenomenon of "fatigue" is becoming simply non-negligible for people living in the present society. According to the results of recent surveys, nearly 60% of the Japanese people responded that they have a feeling of fatigue, and slightly less than 40% of them responded that they have a feeling of fatigue which has been continuing for 6 months or longer. Converting this percentage into the working population, nearly 30 million working people are working while they continue to have a feeling of fatigue. Moreover, in cases of severe chronic fatigue, fatigue could disturb their daily lives. In addition, economical loss caused by such fatigue is estimated to be from several hundreds of billion to one trillion yen, including the cost associated with recovery measures.

Fatigue is broadly classified into acute fatigue and chronic fatigue based on its onset. Acute fatigue develops in a unit of several minutes to several hours, and in many cases it recovers after relatively short period of rest. Chronic fatigue is an accumulation of acute fatigue, which takes several days or, in some cases, several weeks to recover, or it even continues 6 months or longer in severe cases.

Fatigue is also classified into muscle fatigue (physical fatigue) and nervous fatigue(mental fatigue) based on the site of fatigue onset. From the viewpoint of stress, however, in fact these two kinds of fatigue are constantly and mutually related in a complex manner to develop stress. Therefore, appropriate countermeasures against fatigue have not yet been found to date from such a simple classification mentioned above. Meanwhile, if scientific findings regarding such interrelation are obtained, effective measures for the recovery from fatigue may be constructed.

As a method for the prevention of and recovery from fatigue, various methods have been investigated, including simple stress-relieving methods such as bathing, and therapeutic method including drug administration. These methods aim at the recovery from each of the above-mentioned muscle fatigue and nervous fatigue; however, a method to enable concurrent recovery from both kinds of fatigue has not yet been obtained.

In addition, some of these methods involve administration of a certain kinds of medicaments or supplements. Administration of a medicament requires diagnosis and prescription by a physician, so that it is cumbersome. In contrast, dietary countermeasures including administration of supplements are simple and easy to achieve in a daily life, so that research and development of supplements as well as their product development have been promoted in recent years. The size of supplement market has been steadily increasing; for example, supplements for recovery from fatigue such as citric acid, vitamins, and coenzyme Q10 are marketed even in convenience stores. However, these supplements are not shown to clearly exhibit separate effects for the recovery from muscle fatigue and recovery from nervous fatigue, and in fact, a method of recovery from fatigue that satisfies concurrent recovery from both kinds of fatigue has not yet been obtained to date.

A product known by a name "VAAM", which comprises 17 kinds of amino-acids that are contained in the saliva secreted by hornet larva has been attracting attention mainly from its effect on improving athletic performance. With respect to this "VAAM", for example Patent Literature 1 describes that it also has an effect of recovery from fatigue. In addition, development of various amino-acid compositions derived from this technology has been promoted; for example, Patent Literature 2 discloses an amino acid composition comprising 12 kinds of amino-acids used for recovery from muscle fatigue itself as well as accompanying nervous fatigue such as feeling of lassitude.

Furthermore, Patent Literature 3 discloses the use of valine, leucine and isoleucine known as BCAA, as a fatigue prevention/recovery agent for central nervous system, in addition to their original application of improvement in athletic performance, claiming that they have effects in the prevention of fatigue of the central nervous system (cerebral fatigue) and the recovery from brain fatigue.

However, these conventional amino acid compositions have not been sufficiently investigated in terms of concurrent prevention of muscle fatigue and nervous fatigue, and there were problems in industrial (for preparation) and economical viewpoints, because a large amount of various kinds of amino acids should be combined including hardly-soluble amino acids that were cumbersome in preparation, as well as expensive amino acids.

Meanwhile, from the results of several studies regarding fatigue, it is known that during fatigue, the blood concentration of a certain kind of amino acid decreases and a certain kind of amino acid is taken into a specific tissue. For example, Non Patent Literature 1 discloses that in humans subjected to a long period of load by cycling, amino acids such as proline, glycine and alanine are significantly consumed and their levels decrease.

In addition, Non Patent Literature 2 investigates changes in plasma concentrations of various components depending on presence/absence of supplementation of carbohydrates during exercise. Here, the literature shows that plasma concentrations of glycine, alanine, lysine, threonine, and histidine decrease, and in particular, histidine concentration significantly decreases.

Furthermore, in Non Patent Literature 3 as well, changes in plasma concentrations of various components depending on presence/absence of food supply during exercise are investigated. Here, the rate of decrease in tryptophan concentration is shown to be particularly large.

Although the causative relationship between a decrease in the concentration of each amino acid and the exercise or the phenomena of fatigue has not yet been clarified, it is assumed that these amino acids have some relationship in the cycle of energy metabolism and they are consumed in vivo. Therefore, we may think that by supplementing the amino acids whose concentration has been decreased, muscle fatigue can be recovered. However, simple supplementation of these amino acids alone cannot achieve the recovery from both kinds of fatigue, i.e., muscle fatigue and nervous fatigue. Moreover, it has not yet been known that by supplementing these amino acids prior to muscle fatigue and nervous fatigue, both kinds of fatigue can be prevented and even recovered.

Finally JP 3 128318 (which involves the use of threonine and phenylalanine), WO 2004/028528 (which also involves the use of threonine, phenylalanine, glutamic acid, aspartic acid, serine and methionine), SMRIGA MIRO ET AL.: "Exercise-dependent preference for a mixture of branched-chain amino acids and homeostatic control of brain serotonin in exercising rats", JOURNAL OF NUTRITION, vol. 136, no. 2, February 2006 (2006-02), pages 548S-552S, ISSN : 0022-3166 (which involves the use of branched chain amino acids) and JP 8 198748 (which involves the use of L-threonine, L-phenylalanine and L-glutamine) which all deal with muscle and nervous fatigue can also be mentioned.

### [Citation List]

Patent Literature 1: JP No. 2518692
Patent Literature 2: JP A No. 8-198748
Patent Literature 3: WO 02/034257

Non Patent Literature 1: G. Ahlborg, et al. , The Journal of Clinical Investigation, Vol. 53, April 1974, p. 1080-1090
Non Patent Literature 2: T. L. Bazzare, et al., Journal of the American College of Nutrition, 1992, Vol. 11, No. 5, p. 501-511 Non Patent Literature 3: A. H. Forslund, et al., Am. J. Physiol Endocrinol Metab., 2000, Vol. 278, P. 857-867.

### [Summary of Invention]

### Technical Problem

As mentioned above, conventional amino-acid compositions aim mainly at improving athletic performance, and their anti-fatigue effect and effect of fatigue prevention have not yet been sufficiently investigated. In addition, although there are technologies claiming their effects of recovery from fatigue and fatigue prevention, those also have not yet been sufficiently investigated in terms of prevention of both of muscle fatigue and nervous fatigue, so that this problem is still unresolved. Furthermore, with these conventional amino-acid compositions, large amounts of various kinds of amino acids must be supplied as a composition, which possibly and inevitably increases time, effort and cost in their preparation.

The present inventors have found that, as a result of their strenuous research effort, both the muscle fatigue and nervous fatigue can be sufficiently prevented by constructing an amino acid composition with novel composition and quantitative ratio; the inventors further promoted their research and completed the present invention.

Therefore, an object of the present invention is to provide an anti-fatigue agent which comprises an amino acid composition consisting of specific amounts of specific kinds of amino acids.

### Solution to Problem

Namely, the present invention relates to an anti-fatigue agent which comprises an amino acid composition consisting of:
30-200 parts by weight of proline,
60-140 parts by weight of glycine,
25-260 parts by weight of alanine,
40-130 parts by weight of lysine,
20-75 parts by weight of tryptophan,
15-40 parts by weight of histidine,
3-75 parts by weight of tyrosine,
15-45 parts by weight of arginine,
30-55 parts by weight of valine,
35-60 parts by weight of leucine, and
25-60 parts by weight of isoleucine,
wherein muscle fatigue and nervous fatigue are both prevented concurrently.

In addition, the present invention relates to an anti-fatigue composition, according to claim 2 and 3.

Furthermore, the present invention relates to the above anti-fatigue composition, further comprising trehalose.

In addition, the present invention relates to the above anti-fatigue composition, wherein the muscle fatigue is evaluated by measurement of an amount of activity and the nervous fatigue is evaluated by measurement of a blood biomarker concentration.

Furthermore, the present invention relates to the above anti-fatigue composition, with which in an evaluation by measurement of an amount of activity, the amount of activity of an administration group is 110 or grater relative to that of a non-administration group which is set to be 100, and in an evaluation by measurement of a blood biomarker concentration, the measured biomarker concentration of the administration group is 96 or less relative to that of the non-administration group which is set to be 100, so that said composition is evaluated to have prevention effects of muscle fatigue and nervous fatigue.

### Advantageous Effects of Invention

The composition used in the anti-fatigue agent of the present invention is an amino-acid mixture of novel composition and a quantitative ratio different from those in conventional ones. With this amino-acid composition, a significant effect of fatigue prevention, which had conventionally been insufficient, can be provided. Namely, the present invention for the first time enables to prevent both of muscle fatigue and nervous fatigue concurrently.

In the present invention, an amount of activity and a blood biomarker concentration during fatigue are specifically measured to obtain indices of muscle fatigue and nervous fatigue, respectively. As a result, significant effects on fatigue prevention could be exhibited by the inventive amino acid composition in terms of both evaluation indices.

In addition, according to the present invention, the use of only 11 kinds of amino acids, the number of which is smaller than that in conventional amino-acid compositions, can provide superior fatigue-prevention effect, leading to a decrease in the number of kinds of raw materials required, thereby decreasing the time and effort in its preparation and increasing industrial and economical efficiency.

Furthermore, according to the present invention, a predetermined smaller-than-conventional amount of amino acid enables to provide high fatigue-prevention effect, so that superior effects in terms of industrial and economical aspects can be obtained. Moreover, when the amino acid composition is prepared such that an effect similar to that of conventional ones can be obtained, the amount of each amino acid used is smaller than that of the conventional ones; accordingly, an amount of a drink containing such composition can be reduced if the composition is prepared for this purpose, so that the drink is conveniently used for portable drinks such as sport drinks.

The present inventors focused on proline, glycine, alanine, lysine, tryptophan and histidine, which are amino acids whose blood concentrations reduce during exercise, and investigated to formulate these amino acids in the composition at a predetermined concentration so that these are supplemented in the blood, because these amino acids have some influences on a certain reaction in vivo.

Details of the in vivo reaction of the above amino acids are not sufficiently clarified; however, it is considered as follows: for example, proline is used as an energy source for the muscles, has a fat-burning effect and a function to suppress sympathetic nerve activities; alanine is used as an energy source for the muscles and has a function to promote sympathetic nerve activities; lysine promotes appropriate metabolism of fatty acids, has an effect to improve an ability to concentrate and a function to promote sympathetic nerve activities.

In addition, we focused on tyrosine, arginine, valine, leucine and isoleucine which are amino acids whose blood concentration increases during exercise, and investigated to formulate these amino acids in the composition at a predetermined concentration so that these are supplemented outside of the blood in the body, because these amino acids have some influences on a certain reaction in vivo.

Details of the in vivo reaction of the above amino acids are not sufficiently clarified; however, it is considered as follows: for example, arginine promotes synthesis of proteins in the muscles, and has a function to promote sympathetic nerve activities; valine is an essential constituent contained in the proteins of the muscles, and has a function to promote sympathetic nerve activities; leucine has an effect to improve muscle strength, and has a function to promote sympathetic nerve activities; isoleucine exists in a large amount in the proteins of the muscles, is used as an energy source of the muscles, and has a function to suppress sympathetic nerve activities.

Then, we classified tyrosine, arginine, valine, leucine and isoleucine into two groups: branched amino acids (BCAA) such as valine, leucine and isoleucine, and those other than BCAA such as tyrosine and arginine.

### [Best Mode for Carrying out Invention]

Hereinafter, the present invention is explained in detail; however, the present invention is not limited to individual embodiments described below.

As an amino acid composition contained in the anti-fatigue agent of the present invention, those comprising the following amino acids with the following quantitative ratios are preferred. Namely, the anti-fatigue agent which comprises an amino acid composition consisting of 11 kinds of amino acids with the predetermined quantitative ratios is preferred.
30-200 parts by weight of proline;
60-140 parts by weight of glycine;
25-260 parts by weight of alanine;
40-130 parts by weight of lysine;
20-75 parts by weight of tryptophan;
15-40 parts by weight of histidine;
and
3-75 parts by weight of tyrosine;
15-45 parts by weight of arginine;
and
30-55 parts by weight of valine;
35-60 parts by weight of leucine;
25-60 parts by weight of isoleucine.

As an amino acid composition contained in the anti-fatigue agent of the present invention, those comprising the following amino acids with the following quantitative ratios are more preferred. Namely, the anti-fatigue agent which comprises an amino acid composition consisting of 11 kinds of amino acids with the predetermined quantitative ratios is preferred.
30-200 parts by weight of proline;
60-140 parts by weight of glycine;
50-260 parts by weight of alanine;
50-130 parts by weight of lysine;
30-75 parts by weight of tryptophan;
20-40 parts by weight of histidine;
and
3-75 parts by weight of tyrosine;
15-45 parts by weight of arginine;
and
30-55 parts by weight of valine;
35-60 parts by weight of leucine;
25-60 parts by weight of isoleucine.

As an amino acid composition contained in the anti-fatigue agent of the present invention, those comprising the following amino acids with the following quantitative ratios are furthermore preferred. Namely, the anti-fatigue agent which comprises an amino acid composition consisting of 11 kinds of amino acids with the predetermined quantitative ratios is further preferred.
30-150 parts by weight of proline;
60-110 parts by weight of glycine;
50-220 parts by weight of alanine;
50-95 parts by weight of lysine;
30-65 parts by weight of tryptophan;
20-35 parts by weight of histidine;
and
3-65 parts by weight of tyrosine;
20-45 parts by weight of arginine;
and
30-50 parts by weight of valine;
40-60 parts by weight of leucine;
35-55 parts by weight of isoleucine.

As an amino acid composition contained in the anti-fatigue agent of the present invention, those comprising the following amino acids with the following quantitative ratios are particularly preferred. Namely, the anti-fatigue agent which comprises an amino acid composition consisting of 11 kinds of amino acids with the predetermined quantitative ratios is particularly preferred.
30-100 parts by weight of proline;
60-90 parts by weight of glycine;
100-220 parts by weight of alanine;
50-80 parts by weight of lysine;
30-55 parts by weight of tryptophan;
20-30 parts by weight of histidine;
and
5-60 parts by weight of tyrosine;
20-35 parts by weight arginine;
and
30-45 parts by weight of valine;
45-60 parts by weight of leucine;
40-50 parts by weight of isoleucine.

Here, in the amino acid composition with the above composition, tyrosine may be at 3-30 parts by weight, and furthermore at 5-25 parts by weight.

The amino acid compositions, which are the main ingredient of the anti-fatigue agent of the present invention, are extremely safe and their amounts of administration may be set within a wide range of values. In general, the amount may be appropriately set with consideration given to various factors including administration route, and age, body weight and symptoms of a subject of administration including humans. In the present invention, the amount of an amino acid composition as a main ingredient to be administeredmay be set in the range of preferably 0.01-8 g/kg/day, more preferably 0.05-8 g/kg/day, furthermore preferably 0.1-8 g/kg/day, still more preferably 0.3-8 g/kg/day, particularly preferably 0.4-5 g/kg/day, and most preferably 0.5-3 g/kg/day.

Meanwhile, the anti-fatigue agent of the present invention has a characteristic that a smaller amount or a lower concentration of the amino acid composition as the main ingredient can exhibit sufficient effects, as shown by the results of a human test in the later-described examples; therefore, considering the application, effect/efficacy, production cost, etc., the amount of an amino acid composition to be administered may be in the range of preferably 0.01-0.3 g/kg/day, more preferably 0.015-0.28 g/kg/day, furthermore preferably 0.02-0.25 g/kg/day, still more preferably 0.05-0.25 g/kg/day, particularly preferably 0.05-0.2 g/kg/day, and most preferably 0.05-0.15 g/kg/day.

The composition of the present invention may not only be used as an anti-fatigue agent by its prior administration when fatigue prevention is intended, but also be used as a fatigue-recovery agent by subsequent administration when one feels fatigue. Moreover, the anti-fatigue agent of the present invention may be administered via both oral and parenteral (intramuscular, subcutaneous, intravenous, suppository, transdermal, etc.) routes.

As an anti-fatigue agent of oral administration, for example it may be administered with 0.3-30 g 1-3 times/day. In the case of liquid agent, it may be administered in a solution of 0.3-6.0% by weight with 100-500 ml 1-3 times/day. In the case of injection agent by transvenous administration, etc., for example, it may be administered in a solution of 0.3-6.0% by weight with 100-400 ml/day, preferably 150-300 ml/day.

The anti-fatigue agent of the present invention is evaluated to have anti-fatigue effects in terms of both muscle fatigue and nervous fatigue by 2 kinds of evaluation systems, i.e., measurement of amounts of activity during fatigue and measurement of blood biomarker concentrations during fatigue.

Evaluation by the measurement of amounts of activity is carried out as follows, for example. In accordance with the later-described measurement method of amounts of activity in mouse, mice of two groups, i.e., an administration group (mice are administered with an amino acid composition) and a non-administration group (control group), are subjected to physical load by means of a treadmill, etc., and amounts of activity of the two groups are measured; then the relative amount of activity of the administration group is calculated by setting the amount of activity of the non-administration group to be 100. When the amount of activity of the administration group (relative amount) is grater than 100, preferably 110 or grater, more preferably 120 or grater, particularly preferably 190 or grater, then the amino acid composition administered can be evaluated to have a preventive effect of muscle fatigue. Here, we can evaluate that larger the amount of activity of the administration group (relative amount), the higher the preventive effect of muscle fatigue.

Evaluation by the blood biomarker measurement is carried as follows, for example. In accordance with the later-described measurement method of blood biomarker concentrations in mouse, mice of two groups, i.e., an administration group (mice are administered with an amino acid composition) and a non-administration group (control group), are subjected to physical load by means of a treadmill, etc., and their blood is collected after a certain period of time and concentrations of biomarkers in the blood of the two groups are measured; then the relative concentration of the administration group is calculated by setting the measured concentration of the non-administration group to be 100. When any of the blood biomarkers (including cortisol, interferon-γ (IFN-γ), ineterleukin-10 (IL-10)) is used as an index and when the measured concentration of the index in the administration group (relative value) is less than 100, preferably 96 or less, then the agent can be evaluated to have a preventive effect of nervous fatigue.

In particular, when cortisol is used as the index, the agent may be evaluated to have a preventive effect of nervous fatigue when the measured concentration of cortisol in the administration group (relative value) is preferably 95 or less, and more preferably 80 or less. When IFN-γ is used as the index, the agent may be evaluated to have a preventive effect of nervous fatigue when the measured concentration of IFN-γ in the administration group (relative value) is preferably 60 or less, and more preferably 45 or less. When IL-10 is used as the index, the agent may be evaluated to have a preventive effect of nervous fatigue when the measured concentration of IL-10 in the administration group (relative value) is preferably 70 or less, and more preferably 50 or less.

Here, we can evaluate that the smaller the measured concentration of the administration group (relative value), the higher the preventive effect of nervous fatigue.

In the anti-fatigue agent of the present invention, in addition to the above amino acids, the following substances my be comprised if necessary: methionine (preferably 0.3-0.7 mole%, more preferably 0.4-0.6 mole%), asparaginic acid (preferably 0.1-0.3 mole%), taurine (preferably 3 mole% or less), ethanolamine phosphate (preferably 2 mole% or less), cystine (preferably 0.5 mole% or less), β-alaline (preferably 1 mole% or less), γ-aminobutyric acid (preferably 0.5 mole% or less), ornithine or ethanolamine (preferably 3 mole% or less), ammonia (preferably 2 mole% or less), 1-methylhistidine (preferably 3 mole% or less), 3-methylhistidine (preferable 1 mole% or less), etc.

The anti-fatigue agent of the present inventionmay comprise, in addition to the above amino acids, if necessary, components with which anti-fatigue effects may be expected such as citric acid, as well as carnitine, coenzyme Q10, dextrin (linear, branched, cyclic, etc.), vitamins, and minerals.

The amino acids used in the prevent invention are preferably L-amino acids, and it is preferable that each of them alone is of high purity. For example, amino acids with a purity value of those defined in "Japanese Standards of Food Additives" or higher are preferably used. In addition, these amino acids in the form of physiologically acceptable salts may also be used.

The anti-fatigue agent of the present invention preferably comprises trehalose, in particular. Trehalose exists in various animals, plants and microorganisms in the nature; for example it exists in a trophallaxis liquid between hornet and its larva. By combined use of the inventive amino acid composition with trehalose, their synergetic effect can be expected, and the effects of the inventive anti-fatigue agent such as recovery from fatigue and fatigue prevention may be improved. A formulation ratio of trehalose in the inventive anti-fatigue agent is not particularly limited; it may be appropriately set in accordance with its objective and effect. However, the mass ratio of the inventive amino acid composition to trehalose is, for example, preferably 0.45-1.6 : 0.5-5.0, more preferably 0.8-1.6 : 1.0-4.0, and particularly preferably 1.0-1.6 : 1.5-4.0.

Upon production of the inventive amino acid compositions, commercially available above amino acids may be mixed in the above-described predetermined ratios. When the inventive compositions are used as a liquid, this mixture may be dissolved in distilled water or ion-exchange water. Generally, the inventive amino acid composition may be in the form of a powdered homogeneous mixture, which can be dissolved in distilled water or ion-exchange water when necessary. The temperature at which the inventive composition is produced or stored is not particularly limited, but the temperature for production or storage is preferably at room temperature or lower.

The form of administration of the inventive anti-fatigue agent is not particularly limited; the agent may be administered orally or parenterally based on a general method. Specific examples of the form of administration include, for example, formulation into tablet, powder, granule, capsule, syrup, pastille, inhalations, suppository, injection, ointment, eye ointment, eye drops, nasal drops, eardrops, cataplasm, and lotion.

In the above-mentioned formulation process, generally-used excipient, binder, lubricant, coloring agent, and flavoring agent, and if necessary, stabilizer, emulsifier, adsorption enhancing agent, surface active agent, pH adjuster, antiseptic agent and anti-oxidation agent may be used; formulation is carried out by formulating components generally used as raw materials for pharmaceutical preparations by means of routine methods.

Oral preparations are produced as follows, for example: in an amino acid composition according to the present invention or a pharmaceutically acceptable salt thereof, an excipient is added, and if necessary, a binder, a disintegrating agent, a lubricant, a coloring agent, a flavoring agent, etc. are added, then the mixture is formulated into a powder, a fine grain agent, a granule, a tablet, a coated tablet, or a capsule by means of a routine method.

Examples of excipient include lactose, corn starch, white sugar, glucose, mannitol, sorbit, crystalline cellulose, and silicon dioxide. Examples of binder include polyvinyl alcohol, polyvinyl ether, methyl cellulose, ethyl cellulose, gum Arabic, tragacanth, gellatine, shellac, hydroxylpropyl methylcellulose, hydroxylpropyl cellulose, polyvinyl pyrrolidone, polypropylene glycol-polyoxyethylene block polymer, meglumine, etc.

Examples of disintegrating agent include starch, agar-agar, gelatin powder, crystalline cellulose, calcium carbonate, sodium hydrogen carbonate, calcium citrate, dextrin, pectine, carboxymethylcellulose calcium, etc. Examples of lubricant include magnesium stearate, talc, polyethylene glycol, silica, hardened plant oil, etc.

Examples of coloring agent include those which are allowed to be added in medicaments; examples of flavoring agent include cocoa powder, menthol, aromatic powder, peppermint oil, camphor, cinnamic powder, etc. Examples of flavoring agent also include, for example, generally-used sweetening agent, acidulant, flavor, etc.

Liquid agents such as syrup and injection preparations are produced as follows: in an amino acid composition according to the present invention or a pharmaceutically acceptable salt thereof, a pH adjuster, a solubilizer, a tonicity agent, etc. are added, and if necessary, a solubilization-aiding agent and a stabilizer are added, then the mixture is formulated by means of a routine method.

The anti-fatigue agent of the present invention comprises the above amino acid composition and exhibits sufficiently superior effects as medicament; furthermore, with consideration given to palatability (taste), it can be made into food products or specific-purpose food products such as food for specified health use, by appropriately adjusting the concentration of the above amino acid composition. In addition, the anti-fatigue agent of the present invention may be contained in a food composition and is directly taken into the body as a functional food such as a nutritional food, thereby easily providing anti-fatigue effect.

In specific terms, when used as a food composition, the inventive anti-fatigue agent with its original composition may be added into various foods and drinks (milk, soft drink, fermented milk, yogurt, cheese, bread, biscuit, cracker, pizzacrust, formula milk, liquid diet, invalid diet, dry milk for infant, dry milk for breast-feeding women, nutritional food, etc.), which may be taken into the body. In addition, the inventive agent may be mixed with other food products and food components, so that it can be used in accordance with a routine method for general food compositions. With respect to its shape, any generally-used conditions of foods and drinks, for example, solid (powder, granule, etc.), gel, paste, liquid and suspension states may be adopted.

When the inventive agent is used as a food composition, other components may be contained in the above food composition without particular restriction. For example, in foods and drinks, water, protein, glucide, lipid, vitamins, minerals, organic acid, organic base, fruit juice, flavors may be used as ingredients. Examples of protein include animal and plant proteins such as dry whole milk, nonfat dry milk, partially-nonfat dry milk, casein, whey powder, whey protein, whey protein concentrate, whey protein isolate, α-casein, β-casein, κ-casein, β-lactoglobulin, α-lactoalbumin, lactoferrin, soy protein, hen egg protein, meat protein, etc. and their hydrolysates, and various milk-derived components such as butter, whey mineral, cream, whey, nonprotein nitrogen, sialic acid, phospholipid, lactose, etc. Examples of glucide include various sugars, processed starch (in addition to dextrin, solublestarch, britishstarch, acidstarch, starchester, starch ether, etc.) and food fibers. Examples of lipid include animal oil and fat such as lard and fish oil, and their fractionated oil, hydrogenated oil, and ester-exchange oil, as well as vegetal oils such as palm oil, safflower oil, corn oil, rape seed oil and coconut oil, and their fractionated oil, hydrogenated oil, and ester-exchange oil. Examples of vitamins include vitamin A, carotins, vitamin B group, vitamin C, vitamin D group, vitamin E, vitamin K group, vitamin P, vitamin Q, niacin, nicotinic acid, pantothenic acid, biotin, inositol, coline, folic acid, etc. Examples of minerals include calcium, potassium, magnesium, sodium, copper, iron, manganese, zinc and selenium, etc. Examples of organic acid include malic acid, citric acid, lactic acid, tartaric acid, etc. A combination of two or more kinds of these components may be used, and synthetic products and/or foods comprising large amounts of these may also be used.

Hereinafter, the present invention is explained in detail with reference to examples; however, the invention is not limited to these specific examples, and various changes can be made within the range of the claims of the present invention.

### [Examples 1-4 and comparative example 1]

Mice (male, 8monthold, C57BL/6N, breeding condition: 23±3°C, in a cycle of 12-hr bright and 12-hr dark) were divided into two groups, i.e., a sample group (Examples) and a control group (Comparative example), with each group consisting of 3-4 mice such that the average body weights of the two groups become similar. These mice were not subjected to fasting, but were forcibly and orally given an anti-fatigue agent of the present invention (sample) or a physiological saline (control). One hour after the administration, the mice were subjected to physical load by a treadmill test (25 m/min, 60 min).

Each sample with an amount of 500 mg/kg body weight (a suspension (5% by weight) at 10 µL/g body weight) was administered to the mice. Table 1 shows the composition of the amino acids in the anti-fatigue agents of the present invention administered (values of the composition of the amino acids were expressed by parts by weight).

### (Experiment 1: Measurement of amount of activity during fatigue)

After application of the above physical load by a treadmill, amounts of activity were evaluated. Following the completion of the treadmill test, a distance of spontaneous activity (moving distance) during 30 minutes under the light of a red lamp was measured for the sample group (Examples) and the control group (Comparative example). Then, setting the moving distance of the control group as 100, the relative moving distance of the sample group was defined as the amount of activity. Namely, when the amount of activity is large, then the degree of muscle fatigue is considered to be small. Table 1 shows results of the measurement.

### (Experiment 2: Measurement of bloodbiomarker concentration during fatigue)

Blood biomarker concentrations of the mice after the above physical load by a treadmill were evaluated. Following the completion of the treadmill test, blood was collected after passage of a certain period of time, and blood concentrations of cortisol, interferon-γ (IFN-γ), and ineterleukin-10 (IL-10) were measured. Then, setting the values of the control group as 100, the respective relative values of the sample group were defined as the immunological indices in blood (blood biomarkers). Namely, when these values are small, then the degree of feeling of fatigue is small, so that the degree of nervous fatigue is considered to be small. Table 1 shows results of the measurement.

These evaluation methods are in accordance with the method published by Prof. Masayasu Inoue, et al. of the Division of Biochemistry and Molecular Metabolism, Faculty of Medicine, Osaka City University at the Conference of the Japanese Biochemical Society in 2005 (title: Analysis of sex difference in immuno-response system during exercise fatigue, Biochemistry, 2005, Vol. 77, p. 1056).
[Table 1]

**Table 1: Results of measurement of amount of activity and blood biomarker concentration during fatigue.**

| Example No. | Example 1 (Comparative) | Example 2 (Comparative) | Example 3 Invention | Example 4 Invention | Comparative example 3 |
|---|---|---|---|---|---|
| Pro | 53.63 | 197.95 | 34.35 | 37.47 | - |
| Gly | 101.74 | 136.83 | 63.06 | 68.82 | - |
| Ala | 256.56 | 52.05 | 166.64 | 181.82 | - |
| Lys | 74.27 | 120.40 | 59.41 | 64.82 | - |
| Trp | 69.18 | 42.85 | 38.70 | 42.23 | - |
| His | 32.85 | 38.14 | 21.06 | 23.00 | - |
| Tyr | - | - | 54.00 | 5.29 | - |
| Arg | - | - | 23.64 | 25.82 | - |
| Val | - | - | 34.94 | 38.12 | - |
| Leu | - | - | 49.76 | 54.29 | - |
| Ile | - | - | 42.65 | 46.53 | - |
| kinds of amino acids | 6 | 6 | 11 | 11 | 0 |
| Amount of activity | 110 | 119 | 285 | 298 | 100 |
| IFN-γ | 56 | 38 | 51 | 38 | 100 |
| Cortisol | 79 | 76 | 50 | 74 | 100 |
| IL-10 | 96 | 61 | 48 | 65 | 100 |

From the evaluation of the amount of activity, we can see that the mice are able to have larger amounts of activity after physical load of treadmill due to the administration of the anti-fatigue agents of Examples 3-4 of the present invention, and that the inventive agents show higher effects to prevent muscle fatigue compared to Comparative example 3. In particular, the amounts of activity after the exercise in said Examples 3 and 4 are significantly high.

Meanwhile, from the evaluation of the blood biomarker concentrations, all the values of cortisol, IFN-γ and IL-10 in Examples 3-4 were significantly lower than those in Comparative example 3 due to the administration of the inventive anti-fatigue agents. Since each of the concentrations of these compounds increases with an increase in the feeling of fatigue, they can be evaluated as an index for nervous fatigue. Namely, in Examples 3-4 of the present invention, we can see that in addition to the effect of preventing muscle fatigue, effect of preventing nervous fatigue is also high. These effects of relieving fatigue (recovery from fatigue) are particularly significant in Examples 3 and 4.

### [Example 5]

### (Experiment 3: Verification test of anti-fatigue effect)

Next, using human subjects, measurement of acceleration plethysmograms, blood test, urine examination, saliva test and physical test were carried out to verify anti-fatigue effects of the inventive anti-fatigue agents. With these examinations, measurement can be performed without applying unnecessary psychological and physical pain to subjects, and objective evaluation of muscle fatigue and nervous fatigue during application of fatigue is possible.

### (1) Subjects

The subjects were healthy adult males and females (12 males and 6 females, total 18 subjects) among volunteers, who were judged to be appropriate for attending the experiment by physicians in charge of the experiment, and who gave their consent to the experiment. The experiment was carried out in accordance with the Helsinki Declaration under the approval of the Institutional Review Board (IRB).

### (2) Experimental diet

Diet used for the experiment includes a mixture of 11 kinds of amino acids (hereinafter, referred to as "test diet") and a control diet (hereinafter, referred to as "placebo diet"). Table 2 shows their nutritional ingredient composition, and Table 3 shows the composition of amino acids.

The test diet is a hard capsule containing 200 mg of a mixture of 11 kinds of amino acids in 1 capsule, and the placebo diet is a hard capsule without the mixture of 11 kinds of amino acids. A sensory test was performed by the IRB to confirm that the test diet and placebo diet were undistinguishable from sensory aspects such as flavor and taste, as well as their packages.
[Table 2]

**Table 2 : Nutritional ingredient composition in experimental diets (per 1 capsule).**

| Calorie and ingredient composition | Test diet | Placebo diet |
|---|---|---|
| Mixture of 11 kinds of amino acids (mg) | 200 | 0 |
| Crystalline cellulose (mg) | 30 | 230 |
| Calorie (kcal) | 0.9 | 0 |
| Protein (g) | 0.2 | 0 |
| Lipid (g) | 0 | 0 |
| Carbohydrate (g) | 0 | 0.2 |
| Sodium (mg) | 0 | 0 |

[Table 3]

**Table 3: Composition of mixture of 11 kinds of amino acids.**

| Example 5 | |
|---|---|
| Amino acid | Composition |
| | (unit: % by weight) |
| L-proline | 5.84 |
| Glycine | 10.72 |
| L-alanine | 28.33 |
| L-lysine hydrochloride | 10.1 |
| L-tryptophan | 6.58 |
| L-histidine | 3.58 |
| L-tyrosine | 9.18 |
| L-arginine | 4.02 |
| L-valine | 5.94 |
| L-leucine | 8.46 |
| L-isoleucine | 7.25 |

### (3) Test method

### A. Experimental design

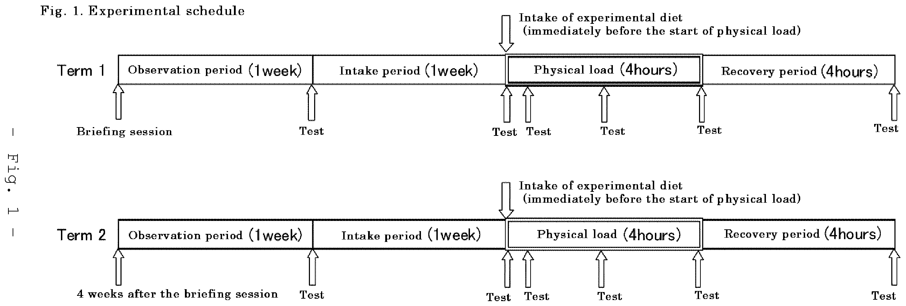

A double-blind test was adopted and a 2-period cross-over experiment using placebo control was performed. The period of experiment was a total of approximately 6 weeks, including 2 cycles of [1 week of observation period, a test day, 1 week of intake period, and a day of physical load] with a 4-week interval between the 2 cycles (refer to Fig. 1). Subjects were instructed to take 5 capsules of experimental diet after breakfast daily for 1 week prior to the physical load and immediately before the start of the physical load. During the entire period of the experiment, the subjects were instructed not to change their daily life habit such as diet and exercise.

### B. The fatigue-inducing physical task consist of workload trial

Using a respiratory metabolic measurement system AE-300S (Minato Medical Science Co., Ltd.) and a cycle ergometer 75XL-IIME (Combi Wellness Corporation), values of anaerobic threshold (AT) were measured prior to the observation period, and the physical work load on the day of physical load was set such that a subject worked on an ergometer for 4 hr (1 hr for 4 times) with a load strength corresponding to 80% of the heart rate at the AT.

Here, 10 min before the start of the physical load, the subjects took 1 bottle (225 ml) of TRELAN G75 (Ajinomoto Pharma Co., Ltd.) for the supply of calorie (75 g as glucose).

### C. Test items

### (a) Acceleration plethysmograms

Accelerated plethysmograms value was measured by an accelerated plethysmograph Artett C (U-Medica Inc.), on the first day of the intake period and the physical load day, prior to the intake of experimental diet, 4 hr after the start of physical load, and after 4 hr of recovery. Two min of measurement was taken for each time.

### (b) Blood test

Blood was collected on the first day of the intake period and the physical load day, prior to the intake of experimental diet, 30 min after the start of physical load (following the high-load test of 10 s that is performed 30 min after the start of the physical load), 4 hr after the start of physical load, and after 4 hr of recovery; then the blood test was carried out. Test items were concentrations of 41 kinds of amino-acid related substances including 20 kinds of amino acids, and 37 items of blood biochemistry test.

### (c) Urine examination

Whole amount of urine on the day of physical load during 4 hr of loading was collected, then concentrations of 3-methoxy-4-hydroxymandelic acid (MHMA), homovanillic acid, and creatinine in the urine were measured.

### (d) Saliva test

Saliva was collected on the day of physical load, prior to the intake of experimental diet, 2 hr after the start of physical load, 4 hr after the start of physical load, and after 4 hr of recovery; then concentrations of amylase, cortisol, chromogranin A and whole protein in the saliva were measured.

### (e) Physical test

Bloodpressure, pulse rate, body temperature, and body weight were measured on the first day of the intake period and the day of physical load, prior to the intake of experimental diet, 2 hr after the start of physical load, 4 hr after the start of physical load, and after 4 hr of recovery.

### D. Test results

### (a) Measurement of acceleration plethysmograms

Among the measurement items, significant differences with a significance level of 5% or less in the Student's t-test were observed in the following items.

In the test-diet group relative to the placebo group, in terms of absolute value, a significant decrease in pulse rate at prior to intake of experimental diet on the physical load day (0 hr) ; a significant increase in Taz and a significant decrease in e/a at 4 hr after the start of physical load (4 hr) ; a significant decrease in the deviation of senescence of blood vessels, significant increases in waveform index I, d/a, coefficient of variation of a-a interval, standard deviation of a-a interval, and range of variation of a-a interval after 4 hr of recovery (8 hr) were observed. In addition, in terms of changes from values prior to the intake, a significant decrease in the amount of change in pulse rate and a significant increase in the amount of change in Taa (at 0 hr relative to prior to the intake), a significant suppression in the reduction of Taz and a significant increase in the amount of change in Tab (at 4 hr relative to prior to the intake) were observed. Furthermore, in terms of changes during physical load, a significant suppression in the increase of deviation of senescence of blood vessels, a significant suppression in the decrease of Waveform index I and a significant decrease in the amount of change in Taz (at 8 hr relative to 4 hr) were observed.

In the frequency analysis, in the test diet group relative to the placebo group, in terms of absolute value, a significant increase in LF%-MEM on the first day of intake (prior to intake), significant increases in LF-FFT and LF%-FFT before intake of experimental diet on the day of physical load (0 hr), a significant decrease in LF%-MEM, and significant increases in LF-FFT and HF-MEM at 4 hr after the start of physical load (4 hr), significant increases in LF-FFT, Total P-MEM and Total P-FFT after 4 hr of recovery (8 hr) were observed. In terms of changes from values prior to the intake, a significant decrease in the amount of change in LF%-MEM (at 4 hr relative to prior to the intake) was observed. In addition, in terms of changes during physical load, a significant suppression in the increase of LF%-MEM, and a significant decrease in the amount of change in LF%-FFT (at 4 hr relative to 0 hr) were observed.

Among the above measurement items in which significant differences were observed, measurement results (absolute values) of d/a, coefficient of variation of a-a interval, and LF%-MEM are shown in Table 4.
[Table 4]

**Table 4: Results of measurement of accelerated plethysmograms.**

| Accelerated plethysmograms (absolute value) | | | | | |
|---|---|---|---|---|---|
| Item | Group | Prior to intake | 0 hr | 4 hr | 8 hr |
| d/a | Placebo group | -0.275±0.104 | -0.272±0.104 | -0.093±0.087 | -0.246±0.222 * |
| | Test-diet group | -0.286±0.132 | -0.276±0.125 | -0.101±0.087 | -0.178±0.118 * |
| Coeffic ient of variati on of a-a interval | Placebo group | 4.71±1.65 | 4.09±1.47 | 4.04±1.83 | 2.65±0.78* |
| | Test-diet group | 4.48±1.58 | 4.29±1.46 | 4.46±2.17 | 3.09±0.91* |

| Frequency analysis (absolute value) | | | | | |
|---|---|---|---|---|---|
| LF%-MEM | Placebo | 34.7±14.8* | 32.4±12.2 | 51.7±18.5* | 43.4±14.7 |
| | group | | | | |
| | Test-di et group | 45.6±15.3* | 41.2±15.2 | 44.6±16.4* | 41.7±16.8 |

| | | | | | |
|---|---|---|---|---|---|
| Mean ± deviation Paired Student's t-test: *p<0.05 | | | | | |

In the above measurement results, d/a is an index that decreases reflecting peripheral vascular resistance, and because this index increases with a vasodilatory action of parasympathetic nerves, it is considered that the test diet suppresses an decrease in the action of parasympathetic nerves, thereby regulating the function of autonomic nerves.

In addition, the coefficient of variation of a-a interval is an index reflecting the action of parasympathetic nerves and is known to decrease with the application of physical-fatigue load; therefore, it is considered that the test diet suppresses a decrease in the action of parasympathetic nerves due to fatigue, thereby regulating the function of autonomic nerves.

Furthermore, LF%-MEM is an index reflecting the action of sympathetic nerves, and because a significant suppression in the increase of LF%-MEM was observed, it is considered that the test diet suppresses an increase in the action of sympathetic nerves, thereby regulating the function of autonomic nerves.

### (b) Blood test

Of the measurement items, significant differences with a significance level of 5% or less in the Student's t-test were observed in the following items.

In the test-diet group relative to the placebo group, in terms of absolute value, a significant decrease in uric acid on the first day of intake (prior to the intake), and significant decreases in triglyceride, sodium, and chlorine at 4 hr after the start of physical load (4 hr) were observed. In addition, in terms of changes from values prior to the intake, a significant increase in the amount of change in uric acid value and a significant decrease in the amount of change in blood glucose level (at 0 hr relative to prior to the intake), a significant increase in the amount of change in uric acid value and a significant suppression in the increase of triglyceride (at 4 hr relative to prior to the intake), and a significant increase in the amount of change in uric acid value (8 hr relative to prior to the intake) were observed. Furthermore, in terms of changes during physical load, a significant decrease in the amount of change in the number of monocytes (at 4 hr relative to 0 hr), significant decreases in the amount of change in alkaline phosphatase and calcium, a significant increase in the amount of change in free carnitine (at 8 hr relative to 0 hr), and a significant increase in the amount of change in chlorine (at 8 hr relative to 4 hr) were observed.

In the amino-acid analysis, in the test-diet group relative to the placebo group, in terms of absolute value, a significant increase in proline/whole albumin ratio on the first day of intake (prior to the intake), significant increases in tyrosine/whole amino acid ratio and aromatic amino acid/whole amino acid ratio at 0.5 hr after the start of physical load (0.5 hr), significant increases in tyrosine and in tyrosine/whole amino acid ratio at 4 hr after the start of physical load (4 hr), and significant increases in tyrosine/whole amino acid ratio and aromatic amino acid/whole amino acid ratio after 4 hr of recovery (8 hr) were observed. In addition, in terms of changes from values prior to the intake, significant suppressions in the increases of tryptophan/whole amino acid ratio and tryptophan/LNAA ratio (at 8 hr relative to prior to the intake) were observed. Furthermore, in terms of changes during physical load, significant increases in the amount of change in lysine/whole amino acid ratio and in the amount of change in taurine (at 4 hr relative to 0 hr), a significant suppression in the increase of cystine (at 8 hr relative to 0 hr), and significant increases in the amount of change in cystine and phenylalanine/whole amino acid ratio and a significant decrease in the amount of change in taurine (at 8 hr relative to 4 hr) were observed.

Among the above measurement items in which significant differences were observed, measurement results (absolute values) of triglyceride and free lipid concentration are shown in Table 5.
[Table 5]

**Table 5: Measurement results of blood test (absolute value).**

| Item | Group | Prior to intake | 0 hr | 4 hr | 8 hr |
|---|---|---|---|---|---|
| TG (mg/dL) | Placebo group | 89±47 | 94±56 | 113±68* | 79±51 |
| | Test-diet group | 93±53 | 83±41 | 95±57* | 66±42 |
| NEFA (mEq/L) | Placebo group | 345±105 | 305±91 | 1288±264* | 50±34 |
| | Test-diet group | 352±120 | 349±127 | 1367±309* | 53±36 |

| | | | | | |
|---|---|---|---|---|---|
| Mean ± deviation Paired Student's t-test: *p<0.05 | | | | | |

In the above measurement results, since a significant decrease in triglyceride and a significant increase in free fatty acid were observed, it is considered that the test diet promotes the decomposition of stored fat into free fatty acid used as an energy source, thereby regulating the energy supply corresponding to a change in the energy metabolism accompanying with an increase in the muscle load.

### (c) Urine test

No significant differences were observed in the Student's t-test with a significance level of 5% or less in the test-diet group, compared to the placebo group.

### (d) Saliva test

Of the measurement items, significant differences with a significance level of 5% or less in the Student's t-test were observed in the following items.

In the test-diet group relative to the placebo group, in terms of absolute value, a significant decrease in amylase at 4 hr after the start of physical load (4 hr), significant decreases in amylase and saliva protein after 4 hr of recovery (8 hr) were observed. In addition, in terms of changes during physical load, a significant suppression in the increase of amylase (at 4 hr relative to 0 hr), and significant suppressions in the increases of amylase and saliva protein (at 8 hr relative to 0 hr) were observed.

Among the above measurement items in which significant differences were observed, measurement results of amylase (AMY) (absolute values and amounts of change) are shown in Table 6.
[Table 6]

**Table 6: Measurement result of saliva test.**

| Saliva test (absolute value) | | | | | |
|---|---|---|---|---|---|
| Item | Group | 0 hr | 2 hr | 4 hr | 8 hr |
| AMY (KU/L) | Placebo group | 28.1±13.0 | 59.0±29.0 | 87.0±35.9* | 79.4±41.9* |
| | Test-diet group | 27.2±15.3 | 56.3±32.0 | 74.3±36.3* | 63.4±36.0* |

| Saliva test (amount of change: a change during physical load) | | | | | |
|---|---|---|---|---|---|
| Item | Group | Amount of change (2vs.0hr) | Amount of change (4vs.0hr) | Amount of change (8vs.0hr) | Amount of change (8vs.4hr) |
| AMY (KU/L) | Placebo group | 31.0±21.0 | 59.0±27.5* | 51.4±34.7* | -7.6±24.9 |
| | Test-diet group | 29.1±25.3 | 47.2±30.0* | 36.2±30.3* | -10.9±21.7 |

| | | | | | |
|---|---|---|---|---|---|
| Mean ± deviation Paired Student's t-test: *p<0.05 | | | | | |

In the above measurement results, an amylase concentration in the saliva is an index reflecting the action of sympathetic nerves; because a significant suppression in the increase of amylase concentration was observed, it is considered that the test diet suppresses an increase in the action of sympathetic nerves, thereby regulating the function of autonomic nerves.

### (e) Physical test

Of the measurement items, significant differences with a significance level of 5% or less in the Student's t-test were observed in the following items.

In the test-diet group relative to the placebo group, in terms of absolute value, a significant increase in body temperature on the first day of intake (prior to the intake) was observed. In addition, in terms of changes from values prior to the intake, significant decreases in the amount of change in pulse rate and in the amount of change in body temperature (at 0 hr relative to prior to the intake), a significant decrease in the amount of change in diastolic pressure (at 2 hr relative to prior to the intake), and a significant suppression in the increase of body temperature (at 8 hr relative to prior to the intake) were observed. Furthermore, in terms of changes during physical load, a significant decrease in the amount of change in diastolic pressure (at 2 hr relative to 0 hr) was observed.

From the above results shown in Tables 4 and 6, we can speculate that the inventive anti-fatigue agent of Example 5 regulates the function of autonomic nerves, alleviates the psychological feeling of stress in subjects, and shows effects in the prevention of and recovery from nervous fatigue, during physical load and during recovery after the physical load.

From the above results shown in Table 5, we can speculate that the inventive anti-fatigue agent of Example 5 promotes burning of fat during physical load, increases the supply of free fatty acid used as an energy source, and shows effects in the prevention of and recovery from muscle fatigue in subjects.

Moreover, in the verification test of the above anti-fatigue effects using humans as subjects, although the amount of intake of the inventive amino-acid composition of Example 5 during the period of intake was as low as only 1000 mg/day (i.e., 15.3 mg/kg/day based on the calculation using the average body weight of the subjects of 65.3 kg), the inventive composition is recognized to have effects in prevention of and recovery from muscle fatigue and nervous fatigue in humans; therefore, if the amount of administration is increased, then further effects may be obtained.

### [Industrial Applicability]

As described above, the anti-fatigue agent comprising the amino acid composition according to the present invention can provide superior anti-fatigue effects of preventing both muscle fatigue and nervous fatigue concurrently. Furthermore, compared to conventional amino acid compositions which aim at improving athletic performance and others, the inventive composition comprises a fewer kinds of amino acids; therefore, the number of kinds of raw materials required for its preparation decreases, leading to superior effects in industrial and economical aspects. Accordingly, the inventive composition has high industrial utility particularly in the field of functional amino acid compositions.

### [Brief Description of Drawings]

[Fig. Figure 1 is a diagram showing the experimental schedule of the verification test of anti-fatigue effect.

## Claims

1. An anti-fatigue composition which comprises an amino acid composition consisting of:
30-200 parts by weight of proline,
60-140 parts by weight of glycine,
25-260 parts by weight of alanine,
40-130 parts by weight of lysine,
20-75 parts by weight of tryptophan,
15-40 parts by weight of histidine,
3-75 parts by weight of tyrosine,
15-45 parts by weight of arginine,
30-55 parts by weight of valine,
35-60 parts by weight of leucine, and
25-60 parts by weight of isoleucine,
wherein muscle fatigue and nervous fatigue are both prevented concurrently.

2. The anti-fatigue composition according to claim 1, which comprises an amino acid composition consisting of:
30-200 parts by weight of proline,
60-140 parts by weight of glycine,
50-260 parts by weight of alanine,
50-130 parts by weight of lysine,
30-75 parts by weight of tryptophan,
20-40 parts by weight of histidine,
3-75 parts by weight of tyrosine,
15-45 parts by weight of arginine,
30-55 parts by weight of valine,
35-60 parts by weight of leucine, and
25-60 parts by weight of isoleucine,
wherein muscle fatigue and nervous fatigue are both prevented concurrently.

3. The anti-fatigue composition according to claim 1 or 2, which comprises an amino acid composition consisting of:
30-150 parts by weight of proline,
60-110 parts by weight of glycine,
50-220 parts by weight of alanine,
50-95 parts by weight of lysine,
30-65 parts by weight of tryptophan,
20-35 parts by weight of histidine,
3-65 parts by weight of tyrosine,
20-45 parts by weight of arginine,
30-50 parts by weight of valine,
40-60 parts by weight of leucine, and
35-55 parts by weight of isoleucine,
wherein muscle fatigue and nervous fatigue are both prevented concurrently.

4. The anti-fatigue composition according to any one of claims 1-3 further comprising trehalose.

## Patentansprüche

1. Anti-Ermüdungs-Zusammensetzung, die eine Aminosäurezusammensetzung aufweist, bestehend aus:
30-200 Gewichtsteilen Prolin,
60-140 Gewichtsteilen Glycin,
25-260 Gewichtsteilen Alanin,
40-130 Gewichtsteilen Lysin,
20-75 Gewichtsteilen Tryptophan,
15-40 Gewichtsteilen Histidin,
3-75 Gewichtsteilen Tyrosin,
15-45 Gewichtsteilen Arginin,
30-55 Gewichtsteilen Valin,
35-60 Gewichtsteilen Leucin, und
25-60 Gewichtsteilen Isoleucin,
wobei Muskelermüdung und Nervenermüdung gleichzeitig verhindert oder vorgebeugt werden.

2. Anti-Ermüdungs-Zusammensetzung nach Anspruch 1, die eine Aminosäurezusammensetzung aufweist, bestehend aus: 30-200 Gewichtsteilen Prolin,
60-140 Gewichtsteilen Glycin,
50-260 Gewichtsteilen Alanin,
50-130 Gewichtsteilen Lysin,
30-75 Gewichtsteilen Tryptophan,
20-40 Gewichtsteilen Histidin,
3-75 Gewichtsteilen Tyrosin,
15-45 Gewichtsteilen Arginin,
30-55 Gewichtsteilen Valin,
35-60 Gewichtsteilen Leucin, und
25-60 Gewichtsteilen Isoleucin,
wobei Muskelermüdung und Nervenermüdung gleichzeitig verhindert oder vorgebeugt werden.

3. Anti-Ermüdungs-Zusammensetzung nach Anspruch 1 oder 2, die eine Aminosäurezusammensetzung aufweist, bestehend aus: 30-150 Gewichtsteilen Prolin,
60-110 Gewichtsteilen Glycin,
50-220 Gewichtsteilen Alanin,
50-95 Gewichtsteilen Lysin,
30-65 Gewichtsteilen Tryptophan,
20-35 Gewichtsteilen Histidin,
3-65 Gewichtsteilen Tyrosin,
20-45 Gewichtsteilen Arginin,
30-50 Gewichtsteilen Valin,
40-60 Gewichtsteilen Leucin, und
35-55 Gewichtsteilen Isoleucin,
wobei Muskelermüdung und Nervenermüdung gleichzeitig verhindert oder vorgebeugt werden.

4. Anti-Ermüdungszusammensetzung nach einem der Ansprüche 1 bis 3, die ferner Trehalose aufweist.

## Revendications

1. Composition anti-fatigue qui comprend une composition d'acides aminés constituée de :
30 à 200 parties en poids de proline,
60 à 140 parties en poids de glycine,
25 à 260 parties en poids d'alanine,
40 à 130 parties en poids de lysine,
20 à 75 parties en poids de tryptophane,
15 à 40 parties en poids d'histidine,
3 à 75 parties en poids de tyrosine,
15 à 45 parties en poids d'arginine,
30 à 55 parties en poids de valine,
35 à 60 parties en poids de leucine, et
25 à 60 parties en poids d'isoleucine,
permettant de prévenir ou d'empêcher simultanément la fatigue musculaire et la fatigue nerveuse.

2. Composition anti-fatigue selon la revendication 1, qui comprend une composition d'acides aminés constituée de :
30 à 200 parties en poids de proline,
60 à 140 parties en poids de glycine,
50 à 260 parties en poids d'alanine,
50 à 130 parties en poids de lysine,
30 à 75 parties en poids de tryptophane,
20 à 40 parties en poids d'histidine,
3 à 75 parties en poids de tyrosine,
15 à 45 parties en poids d'arginine,
30 à 55 parties en poids de valine,
35 à 60 parties en poids de leucine, et
25 à 60 parties en poids d'isoleucine,
permettant de prévenir ou d'empêcher simultanément la fatigue musculaire et la fatigue nerveuse.

3. Composition anti-fatigue selon la revendication 1 ou 2, qui comprend une composition d'acides aminés constituée de :
30 à 150 parties en poids de proline,
60 à 110 parties en poids de glycine,
50 à 220 parties en poids d'alanine,
50 à 95 parties en poids de lysine,
30 à 65 parties en poids de tryptophane,
20 à 35 parties en poids d'histidine,
3 à 65 parties en poids de tyrosine,
20 à 45 parties en poids d'arginine,
30 à 50 parties en poids de valine,
40 à 60 parties en poids de leucine, et
35 à 55 parties en poids d'isoleucine,
permettant de prévenir ou d'empêcher simultanément la fatigue musculaire et la fatigue nerveuse.

4. Composition anti-fatigue selon l'une quelconque des revendications 1 à 3 comprenant en outre du tréhalose.
